# EUROPEAN PATENT APPLICATION

(11) **EP 1 930 023 A2**
(43) Date of publication of application: **11.06.2008**
(21) Application number: 08102543.9
(22) Date of filing: 08.04.2002
(51) Int. Cl.: A61K 38/18, A61K 45/00, A61P 43/00

(54) **Erythropoietin ameliorates chemotherapy-induced toxicity in vivo**

(30) Priority: 09.04.2001 US 282621 P
(62) Divisional of application: 02725577.7
(71) Applicant: EAST CAROLINA UNIVERSITY, Greenville, NC 27858-4353 (US)
(72) Inventor: Sigounas, George, Greenville, NC 27858 (US); Mehlhop, Paul, Winterville, NC 28590 (US)
(74) Representative: Harrison Goddard Foote

(57) **Abstract**

A method of reducing organ toxicity in a subject being administered a cytotoxic agent comprising concurrently administering with the cytotoxic agent erythropoietin (EPO), the EPO being administered in an amount effective to reduce pulmonary toxicity caused by the cytotoxic agent.

## Description

### Related Applications

This application claims the benefit of provisional application serial number 60/282,621 filed April 9,2001, the disclosure of which is incorporated by reference herein in its entirety.

### Field of the Invention

The present invention concerns methods of reducing organ cytotoxicity such as pulmonary toxicity in a subject being administered a cytotoxic agent such as an antineoplastic agent.

### Background of the Invention

Erythropoietin (EPO) is a glycoprotein produced in the kidney, and is the principal hormone responsible for stimulating red blood cell production (erythrogenesis). EPO stimulates the division and differentiation of committed erythroid progenitors in the bone marrow. Normal plasma erythropoietin levels range from 0.01 to 0.03 Units/mL, and can increase up to 100 to 1,000-fold during hypoxia or anemia. Graber and Krantz, Ann. Rev. Med. 29:51 (1978); Eschbach and Adamson, Kidney Intl. 28:1 (1985). Recombinant human erythropoietin (rHuEpo or epoetin alfa) is commercially available as Epogen® (Amgen Inc., Thousand Oaks, CA) and as Procrit® (Ortho Biotech Inc., Raritan, NJ). EPO is frequently used to increase the hematocrit of cancer patients who become anemic because of their disease or because of treatment with chemotherapeutic drugs. EPO is indicated for treatment of anemia, including anemias associated with cancer chemotherapy, chronic renal failure, malignancies, adult and juvenile rheumatoid arthritis, disorders of haemoglobin synthesis, prematurity, and zidovudine treatment of HIV infection.

Pulmonary fibrosis is the most common manifestation of pulmonary toxicity seen with chemotherapeutic drugs. Pulmonary fibrosis is a pathologic deposition of polymerized fibrin in the interstitial spaces. Its precise pathophysiology is not completely understood but it is usually seen in the context of chronic inflammation resulting from exposure to exogenous agents. Although pulmonary damage has been associated with many antineoplastic agents, bleomycin, nitrosoureas, mitomycin, busulfan, ara-C, interleukin-2 and methotrexate are the chemotherapeutic agents most frequently associated with lung toxicity. Thus, there is a need for new ways to reduce organ toxicity associated with administration of a cytotoxic agent.

### Summary of the Invention

According to embodiments of the present invention, the present invention is a method of reducing organ toxicity in a subject being administered a cytotoxic agent, the method comprising concurrently administering with the cytotoxic agent erythropoietin (EPO; the "active agent"), the EPO being administered in an amount effective to reduce organ toxicity caused by the cytotoxic agent. The cytotoxic agent is, in general, administered in an amount intended to achieve its therapeutic effect, such as an antineoplastic amount of an antineoplastic agent.

According to embodiments of the present invention, organs to be treated or protected by the methods of the present invention include liver, brain, kidneys, and lungs, with the lungs being particularly preferred.

According to other embodiments of the present invention, the antineoplastic agent is one that is cytotoxic to the lungs of the subject.

According to particular embodiments, the antineoplastic agent is an antibiotic, a nitrosourea, an alkyl sulfonate, a cytodine analog, a vinca alkaloid, an epipodophylotoxin, an interleukin, a folic acid analog, or combinations thereof.

According to still other embodiments of the present invention, the present invention includes the use of an active agent as described above for the manufacture of a medicament for carrying out a method as described above.

The foregoing and other embodiments and aspects of the present invention are explained in greater detail in the drawings herein and the specification set forth below.

### Brief Description of the Drawings

Figure 1 illustrates the effect of EPO and bleomycin on pulmonary function after treatment for two weeks; and
**Figure 2** illustrates the effect of EPO and bleomycin on pulmonary function after treatment for four weeks.

### Detailed Description of the Preferred Embodiments

Subjects to be treated by the method of the present invention include both human and animal (e.g., dog, cat, cow, horse) subjects, and are preferably mammalian subjects.

As used herein, the administration of at least one compound concurrently with at least one second compound means that the compounds are administered closely enough in time that the presence of one alters the biological effects of the other. The at least two compounds may be administered simultaneously or sequentially. Sequential administration allows administration of compounds effective to reduce chemotherapy-' induced toxicity as a preventive treatment prior to introduction of the cytotoxic agent, or as a treatment to reduce chemotherapy-induced toxicity as a result of previous administration of at least one cytotoxic agent.

***Cytotoxic Agents**.* Cytotoxic agents which may be used in conjunction with the present invention are, in general, antineoplastic agents. As used herein, the term antineoplastic or chemotherapeutic agent refers to agents which preferentially kill neoplastic cells or disrupt the cell cycle of rapidly proliferating cells, used therapeutically to prevent or reduce the growth of neoplastic cells. As used herein, chemotherapy includes treatment with a single chemotherapeutic agent or with a combination of agents. In a subject in need of treatment, chemotherapy may be combined with surgical treatment or radiation therapy, or with other antineoplastic treatment modalities.

Antineoplastic agents include those generally known to those skilled in the art. Exemplary antineoplastic agents include, but are not limited to, vinca alkaloids, epipodophyllotoxins, anthracycline antibiotics, actinomycin D, plicamycin, puromycin, gramicidin D, paclitaxel (Taxol®, Bristol Myers Squibb), colchicine, cytochalasin B, emetine, maytansine, arnsacrine (or "mAMSA"), and nitrosoureas. The vinca alkaloid class is described in Goodman and Gilman's The Pharmacological Basis of Therapeutics, 1277-1280 (7th ed. 1985) (hereafter "Goodman and Gilman"). Exemplary vinca alkaloids include, but are not limited to, vincristine, vinblastine, and vindesine. The epipodophyllotoxin class is described in Goodman and Gilman, *supra* at 1280-1281. Exemplary epipodophyllotoxins include, but are not limited to, etoposide, etoposide orthoquinone, and teniposide. The anthracycline antibiotic class is described in Goodman and Gilman, *supra* at 1283-1285. Exemplary anthracycline antibiotics include, but are not limited to, daunorubicin, doxorubicin, mitoxantraone, and bisanthrene. Actinomycin D, also called Dactinomycin, is described in Goodman and Gilman, *supra* at 1281-1283. Plicamycin, also called mithramycin, is described in Goodman and Gilman, *supra* at 1287-1288. Nitrosoureas include, but are not limited to, lomustine [CCNU] (CeeNU®, Bristol Myers Squibb), carmustine [BCNU] (BiCNU®), Bristol Myers Squibb), semustine [methyl-CCNU] and streptozocin.

Additional chemotherapeutic agents include, but are not limited to, cisplatin (Platinol®, Bristol Myers Squibb); carboplatin (Paraplatin®, Bristol Myers Squibb); mitomycin (Mutamycin®, Bristol Myers Squibb); altretamine (Hexalen®, U.S. Bioscience, Inc.); cyclophosphamide (Cytoxan®, Bristol Myers Squibb); busulfan (MYLERAN®, GlaxoSmithKline; cytarabine, such as ara-C, interleukin-2, and methotrexate.

Methods of administering chemotherapeutic drugs vary depending upon the specific agent used, as would be known to one skilled in the art. Depending upon the agent used, chemotherapeutic agents may be administered, for example, by injection (intravenously, intramuscularly, intraperitoneally, subcutaneously, intrathecal, intratumor, intrapleural) or orally.

***Erythropoietin.*** As used herein, human erythropoietin (EPO) refers to both the naturally occurring human erythropoietin glycoprotein as well as recombinant human erythropoietin (rHuEpo or epoetin alfa, available commercially as Epogen® (Amgen Inc., Thousand Oaks, CA) and as Procrit® (Ortho Biotech Inc., Raritan, NJ)). Peptide analogs of EPO may also be used in the methods of the present invention. As used herein, peptide analogs are those compounds which, while not having amino acid sequences identical to that of EPO, have a similar three-dimensional structure. In protein molecules which interact with a receptor, the interaction takes place at the surface-accessible sites in a stable three-dimensional molecule. By arranging the critical binding site residues in an appropriate conformation, peptides which mimic the essential surface features of EPO binding regions may be designed and synthesized in accordance with known techniques. A molecule which has a surface region with essentially the same molecular topology to the binding surface of EPO will be able to mimic the interaction of EPO with the EPO receptor. Methods for determining peptide three-dimensional structure and analogs thereto are known, and are sometimes called "rational drug design techniques." *See, e.g.,* U.S. Patent No. 4,833,092 to Geysen; U.S. Patent No. 4,859,765 to Nestor; U.S. Patent No. 4,853,871 to Pantoliano; U.S. Patent No. 4,863,857 to Blalock (applicants specifically intend that the disclosures of all U.S. patents cited herein be incorporated by reference in their entirety).

Peptides which mimic the biological activity of erythropoietin (EPO receptor peptide ligands) may be substituted for EPO in the methods of the present invention. The sequence of such peptides may represent fragments, of the full-length EPO protein sequence, which fragments are capable of binding to and activating the EPO receptor. Additionally, peptides with sequences dissimilar to that of EPO may be utilized in the methods of the present invention, where such peptides mimic the biological activity of EPO. Wrighton et al. report the identification and characterization of small peptides that bind to and activate the erythropoietin receptor on the surface of target cells, although the peptides' sequences are not similar to the primary sequence of EPO (Wrighton et al., Science 273:458 (26 July 1996)). These peptide agonists are represented by a 14-amino acid disulfide-bonded cyclic peptide with an identified minimum consensus sequence. The structure of a complex of one such peptide mimetic with the erythropoietin receptor is described by Livnah et al., Science 273:464 (26 July 1996).

EPO used according to the methods of the present invention may be administered by any suitable means, as would be apparent to one skilled in the art. EPO may be administered systemically (e.g., intravenously) or locally (e.g., injected into a tumor, tissues immediately surrounding a tumor, or into an anatomic compartment containing a tumor). Where a chemotherapeutic agent is delivered systemically, for example, an endothelial-protecting amount of EPO may be administered systemically by intravenous injection.

The dosage and timing of EPO administration used in conjunction with a chemotherapeutic agent will similarly depend upon the desired effect. The present inventors have discovered that depending upon the timing of EPO administration (simultaneous with, before, or after chemotherapeutic agent administration) and the dosage of EPO, EPO either protects the endothelium from the growth-inhibiting effects of chemotherapeutic agents, or enhances the endothelial growth inhibition seen with chemotherapeutic agents. It will be apparent to those skilled in the art how to determine, by routine experimentation, the dosage and timing of EPO administration in conjunction with a particular chemotherapeutic agent to achieve a desired effect.

The maximum amount of EPO that can be administered in single or multiple doses has not been determined. Doses of up to 1,500 Units/kg for three to four weeks have been administered without toxic effects due to EPO itself. Eschbach et al., in: Prevention of Chronic Uremia (Friedman et al., eds.), Field and Wood Inc., Philadelphia, pp. 148-155 (1989). Suitable dosages may range from about 1, 10, or 100 Units per kilogram (U/kg) to about 200, 1,000 or 2,000 U/kg.

***Pharmaceutical formulations.*** The active compounds described above may be formulated for administration in a pharmaceutical carrier in accordance with known techniques. *See, e.g.,* Remington, The Science And Practice of Pharmacy (9th Ed. 1995). In the manufacture of a pharmaceutical formulation according to the invention, the active compound (including the physiologically acceptable salts thereof) is typically admixed with, *inter alia,* an acceptable carrier. The carrier must, of course, be acceptable in the sense of being compatible with any other ingredients in the formulation and must not be deleterious to the patient. The carrier may be a solid or a liquid, or both, and is preferably formulated with the compound as a unit-dose formulation, for example, a tablet, which may contain from 0.01 or 0.5% to 95% or 99% by weight of the active compound. One or more active compounds may be incorporated in the formulations of the invention, which may be prepared by any of the well known techniques of pharmacy consisting essentially of admixing the components, optionally including one or more accessory ingredients.

The formulations of the invention include those suitable for oral, rectal, topical, buccal (e.g., sub-lingual), vaginal, parenteral (e.g., subcutaneous, intramuscular, intradermal, or intravenous), topical (*i.e*., both skin and mucosal surfaces, including airway surfaces) and transdermal administration, although the most suitable route in any given case will depend on the nature and severity of the condition being treated and on the nature of the particular active compound which is being used.

Formulations suitable for oral administration may be presented in discrete units, such as capsules, cachets, lozenges, or tablets, each containing a predetermined amount of the active compound; as a powder or granules; as a solution or a suspension in an aqueous or non-aqueous liquid; or as an oil-in-water or water-in-oil emulsion. Such formulations may be prepared by any suitable method of pharmacy which includes the step of bringing into association the active compound and a suitable carrier (which may contain one or more accessory ingredients as noted above). In general, the formulations of the invention are prepared by uniformly and intimately admixing the active compound with a liquid or finely divided solid carrier, or both, and then, if necessary, shaping the resulting mixture. For example, a tablet may be prepared by compressing or molding a powder or granules containing the active compound, optionally with one or more accessory ingredients. Compressed tablets may be prepared by compressing, in a suitable machine, the compound in a free-flowing form, such as a powder or granules optionally mixed with a binder, lubricant, inert diluent, and/or surface active/dispersing agent(s). Molded tablets may be made by molding, in a suitable machine, the powdered compound moistened with an inert liquid binder.

Formulations of the present invention suitable for parenteral administration comprise sterile aqueous and non-aqueous injection solutions of the active compound, which preparations are preferably isotonic with the blood of the intended recipient. These preparations may contain anti-oxidants, buffers; bacteriostats and solutes which render the formulation isotonic with the blood of the intended recipient. Aqueous and non-aqueous sterile suspensions may include suspending agents and thickening agents. The formulations may be presented in unit\dose or multi-dose containers, for example sealed ampoules and vials, and may be stored in a freeze-dried (lyophilized) condition requiring only the addition of the sterile liquid carrier, for example, saline or water-for-injection immediately prior to use. Extemporaneous injection solutions and suspensions may be prepared from sterile powders, granules and tablets of the kind previously described. For example, in one aspect of the present invention, there is provided an injectable, stable, sterile composition comprising an active compound, or a salt thereof, in a unit dosage form in a sealed container. The compound or salt is provided in the form of a lyophilizate which is capable of being reconstituted with a suitable pharmaceutically acceptable carrier to form a liquid composition suitable for injection thereof into a subject. The unit dosage form typically comprises from about 10 mg to about 10 grams of the compound or salt. When the compound or salt is substantially water-insoluble, a sufficient amount of emulsifying agent which is physiologically acceptable may be employed in sufficient quantity to emulsify the compound or salt in an aqueous carrier. One such useful emulsifying agent is phosphatidyl choline.

In addition to active agents or their salts, the pharmaceutical compositions may contain other additives, such as pH-adjusting additives. In particular, useful pH-adjusting agents include, but are not limited to, acids, such as hydrochloric acid, bases or buffers, such as sodium lactate, sodium acetate, sodium phosphate, sodium citrate, sodium borate, or sodium gluconate. Further, the compositions may contain microbial preservatives. Useful microbial preservatives include, but are not limited to, methylparaben, propylparaben, and benzyl alcohol. The microbial preservative is typically employed when the formulation is placed in a vial designed for multidose use. Of course, as indicated, the pharmaceutical compositions of the present invention may be lyophilized using techniques well known in the art.

The present invention is explained in greater detail in the following non-limiting Examples.

### EXAMPLE 1

### Treatment Protocol

C57B16 female mice between 7 and 12 weeks old weighing approximately 20 grams were treated with varying doses of EPO alone and in combination with bleomycin. Animals were treated twice a week with placebo, EPO alone (5-40 µg/mouse, i.p.), bleomycin alone (0.25 -0.75 µg/mouse, i.p.), or EPO and bleomycin in combination. Animals were given a graded methacholine challenge (0-48 mg/ml) at 2 weeks, 4 weeks, and 6 weeks. At these time points, pulmonary function was measured using a full body plethysmograph (Buxco, Niskayuna, NY). This is a non-invasive method utilizing a clear plastic animal chamber fitted with two pneumotachs and a transducer which interface with a computer. Pulmonary resistance and dynamic compliance is evaluated by an analyzer which measures flow and pressure readings at equivalent lung volumes during inspiration and expiration ("Iso-Volumetric Method"). Artifacts from animal movement or atypical breaths (e.g. from "sniffing") are ignored by computer analysis using parameters that can be adjusted as appropriate for the tidal volume and inspiratory time of the individual animal. The method of whole body plethysmography has been studied and found to be a valid index of pulmonary function in the mouse (Harnelmann et al., Am. J. Respir. Crit. Care Med. 156)3 Pt1 776-75 (1997).

### EXAMPLE 2

### Experimental Results

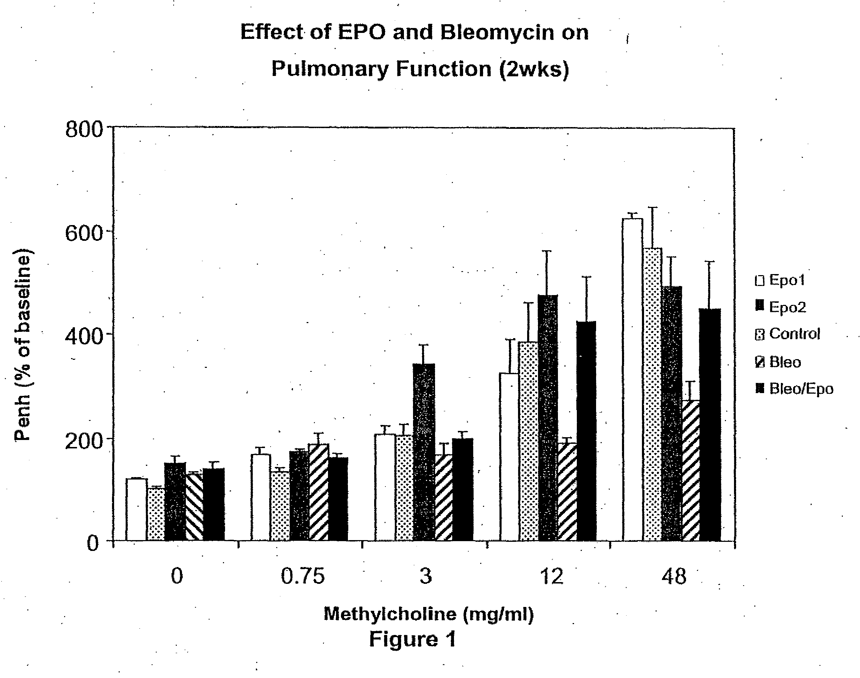

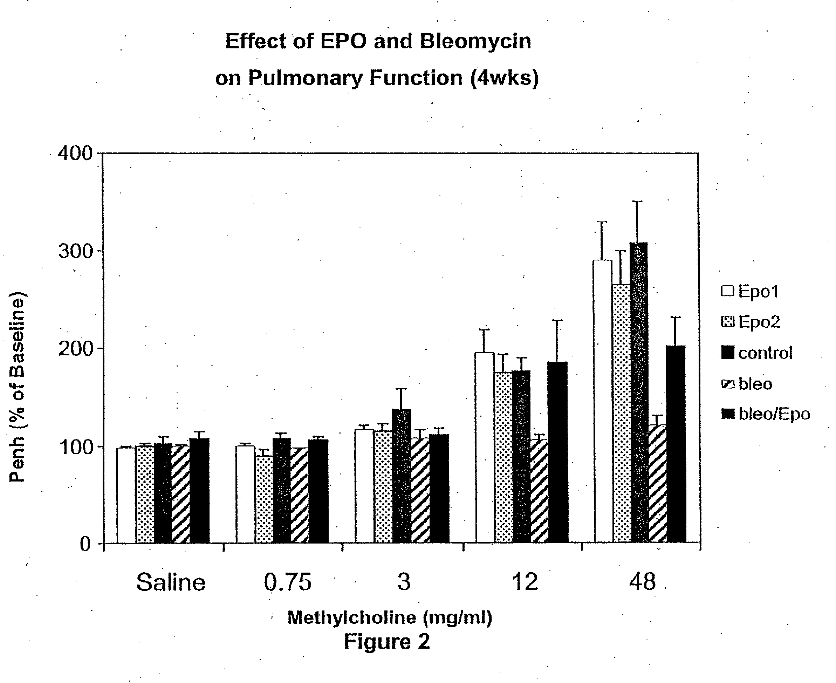

Bleomycin was found to decrease pulmonary function as reflected by changes in the enhanced pause ("Penh", a surrogate for airway resistance). Animals treated twice a week for two weeks with bleomycin and given a graded (0 to 48 mg/ml) methacholine challenge had a 2.6-fold worsening in Penh values compared with control animals (see **Figure 1**). EPO given in the absence of bleomycin induced a slight enhancement of pulmonary function. However, mice treated with a combination of bleomycin and erythropoietin had 60% better pulmonary function than mice treated with bleomycin alone. A similar pattern of the Penh values was observed when pulmonary functions were performed at four weeks of treatment (see **Figure 2**).

The foregoing is illustrative of the present invention, and is not to be construed as limiting thereof. The invention is defined by the following claims, with equivalents of the claims to be included therein.

The invention includes the following recitals as set forth in points 1-17.
1. A method of reducing chemotherapy-induced toxicity in a subject being administered a cytotoxic agent, comprising administering a cytotoxic agent concurrently with erythropoietin in an amount effective to reduce chemotherapy-induced toxicity.
2. The method according to point 1, wherein said cytotoxic agent is an antineoplastic agent.
3. The method according to point 2, wherein said antineoplastic agent is selected from the group consisting of an antibiotic, a nitrosourea, an alkyl sulfonate, a cytodine analog, a vinca alkaloid, an epipodophylotoxin, an interleukin, and a folic acid analog, and combinations thereof.
4. The method according to point 2, wherein said antineoplastic agent is bleomycin, mitomycin, or carmustine, and combinations thereof.
5. The method according to point 1, wherein said chemotherapy-induced toxicity is selected from the group consisting of pulmonary toxicity, liver toxicity, CNS toxicity, and renal toxicity, and combinations thereof.
6. The method according to point 1, wherein said chemotherapy-induced toxicity is pulmonary toxicity.
7. The method according to point 1, wherein said erythropoietin is administered in an amount from about 1 U/kg to about 2000 U/kg.
8. The method according to point 1, wherein said erythropoietin is administered in an amount from about 1U/kg to about 200 U/kg.
9. The method according to point 1, wherein said administering step comprises oral, rectal, topical, buccal, vaginal, parenteral, topical, or transdermal administration, and combinations thereof.
10. A method of reducing pulmonary toxicity in a subject being administered a cytotoxic agent, comprising administering a cytotoxic agent concurrently with erythropoietin in an amount effective to reduce pulmonary toxicity.
11. The method according to point 10, wherein said cytotoxic agent is a antineoplastic agent.
12. The method according to point 11, wherein said antineoplastic agent is cytotoxic to the lungs of said subject.
13. The method according to point 11, wherein said antineoplastic agent is selected from the group consisting of an antibiotic, a nitrosourea, an alkyl sulfonate, a cytodine analog, a vinca alkaloid, an epipodophylotoxin, an interleukin, and a folic acid analog, and combinations thereof.
14. The method according to point 11, wherein said antineoplastic agent is bleomycin, mitomycin, or carmustine, and combinations thereof.
15. The method according to point 10, wherein said erythropoietin is administered in an amount from about 1 U/kg to about 2000 U/kg.
16. The method according to point 10, wherein said erythropoietin is administered in an amount from about 1U/kg to about 200 U/kg.
17. The method according to point 10, wherein said administering step comprises oral, rectal, topical, buccal, vaginal, parenteral, topical, or transdermal administration, and combinations thereof.

## Claims

1. Erythropoietin for the treatment or reduction of organ toxicity induced by a cytotoxic chemotherapeutic agent, the erythropoietin being for administration simultaneously with, before or after the chemotherapeutic agent.

2. Erythropoietin of claim 1 for the treatment or reduction of chemotherapy-induced CNS toxicity.

3. Erythropoietin of claim 1 for the treatment or reduction of chemotherapy-induced kidney toxicity.

4. Erythropoietin of claim 1 for the treatment or reduction of chemotherapy-induced liver toxicity.

5. Erythropoietin of claim 1 wherein the organ does not include lungs.

6. Erythropoietin of claim 1 for the treatment or reduction of chemotherapy-induced pulmonary toxicity.

7. Erythropoietin of any preceding claim wherein the cytotoxic agent is an antineoplastic agent.

8. Erythropoietin of claim 7 wherein the antineoplastic agent is an antibiotic, a nitrosourea, an alkyl sulfonate, a cytodine analog, a vinca alkaloid, an epipodophylotoxin, an interleukin, a folic acid analog, bleomycin, mitomycin or carmustine, or a combination thereof.

9. Erythropoietin of claim 7 wherein the antineoplastic agent is bleomycin, mitomycin or carmustine, or a combination thereof.

10. Erythropoietin of any preceding claim for administration in an amount from about 1 U/kg to about 2000 U/kg.

11. Erythropoietin of any preceding claim for administration in an amount from about 1 U/kg to about 200 U/Kg.

12. A medicament for reducing organ toxicity induced by a cytotoxic agent, the medicament comprising erythropoietin and being for administration simultaneously or sequentially with the cytotoxic agent such that the presence of the erythropoietin alters the biological effect of the cytotoxic agent.

13. A medicament of claim 12 which is a parenteral medicament.

14. A medicament of claim 12 which is an intravenous medicament.

15. A medicament of any of claims 12 to 14 wherein the organ is the CNS.

16. A medicament of any of claims 12 to 14 wherein the organ is the kidney.

17. A medicament of any of claims 12 to 14 wherein the organ is the liver.

18. A medicament of any of claims 12 to 14 wherein the organ does not include lungs.

19. A medicament of any of claims 12 to 14 wherein the organ is the lungs.

20. A medicament of any of claims 12 to 19 wherein the cytotoxic agent is an antineoplastic agent.

21. A medicament of claim 20 wherein the antineoplastic agent is an antibiotic, a nitrosourea, an alkyl sulfonate, a cytodine analog, a vinca alkaloid, an epipodophylotoxin, an interleukin, a folic acid analog, bleomycin, mitomycin or carmustine, or a combination thereof.

22. A medicament of claim 21 wherein the antineoplastic agent is bleomycin, mitomycin or carmustine, or a combination thereof.

23. A medicament of any of claims 12 to 22 which is for administration in a dosage such that the erythropoietin is administered in an amount from about 1 U/kg to about 2000 U/kg.

24. A medicament of claim 23 which is for administration in a dosage such that the erythropoietin is administered in an amount from about U/kg to about 200 U/Kg.
